(19) Europäisches Patentamt / European Patent Office / Office européen des brevets

(11) **EP 4 406 942 A1**

(12) **EUROPEAN PATENT APPLICATION**

(43) Date of publication:
**31.07.2024 Bulletin 2024/31**

(21) Application number: **23153520.4**

(22) Date of filing: **26.01.2023**

(51) International Patent Classification (IPC):
***C07D 277/42*** *(2006.01)* ***A61K 31/426*** *(2006.01)*
***A61P 29/00*** *(2006.01)* ***A61P 35/00*** *(2006.01)*

(52) Cooperative Patent Classification (CPC):
**C07D 277/42; A61P 29/00; A61P 35/00**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Dompe' Farmaceutici S.P.A.**
**20122 Milan (IT)**

(72) Inventors:
- **ARAMINI, Andrea**
  **67100 L'Aquila (IT)**
- **BIANCHINI, Gianluca**
  **67100 L'Aquila (IT)**
- **ALLEGRETTI, Marcello**
  **67100 L'Aquila (IT)**

(74) Representative: **Dompé farmaceutici Spa**
**Via San Martino, 12-12/a**
**20122 Milano (IT)**

# (54) STABLE MONOHYDRATE OF DF2755A AND PROCESS FOR ITS PREPARATION

(57) The present invention relates to a stable monohydrate of compound DF2755A, to a process for its manufacture, to its pharmaceutical compositions and medical uses.

The monohydrate of compound DF2755A of the present invention is physically, chemically and optically stable, thus particularly advantageous for pharmaceutical applications.

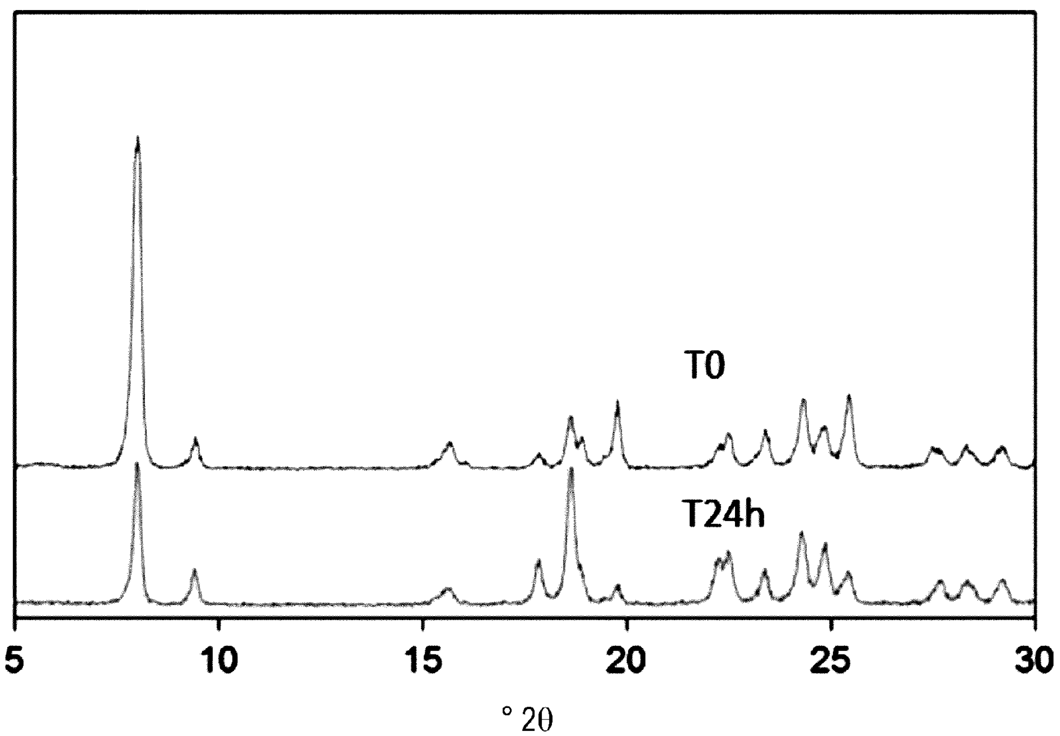


FIG.6

EP 4 406 942 A1

Processed by Luminess, 75001 PARIS (FR)

## Description

FIELD OF THE INVENTION

The present invention relates to the monohydrate of the compound DF2755A, to a process for its manufacture, to its pharmaceutical compositions and medical uses.

BACKGROUND OF THE INVENTION

International patent application WO2010031835A2 in the name of the Applicant, discloses a class of compounds of formula (I):

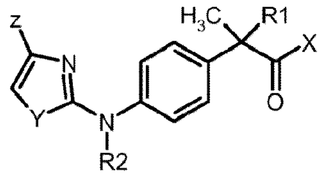

(I)

and their chemical syntheses.

In particular, the above document discloses the compound 2-(4-{[4-(trifluoromethyl)-1,3-thiazol-2-yl]amino}phenyl)propanoic acid (wherein in formula (I) Z is $CF_3$; Y is S; R1, R2 are H) (Example 1), its 2S enantiomer (2S)-2-(4-{[4-(trifluoromethyl)-1 ,3-thiazol-2-yl] amino} phenyl) propanoic acid (Example 3), also known as DF2755Y,

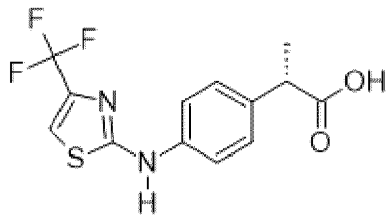

DF2755Y

and the sodium salt thereof, namely sodium (2S)-2-(4-{[4-(trifluoromethyl)-1,3-thiazol-2-yl] amino} phenyl) propanoate (Example 3a) also known as DF2755A:

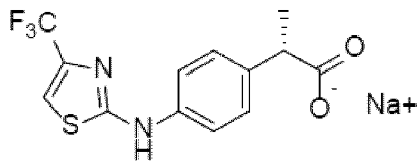

DF2755A

Compound DF2755A appears to be particularly promising as it is characterized by a selective dual potent inhibitory activity of both CXCR1 and CXCR2 receptors and a favorable oral pharmacokinetic profile. The compound thus finds potential therapeutic application in colon, prostate, pancreatic, breast, ovarian cancer, melanoma, inflammatory and post-operative pain, inflammatory-mediated diseases such as bullous pemphigoid, cystic fibrosis, chronic obstructive pulmonary disorder, asthma, psoriasis, rheumatoid arthritis, inflammatory bowel disease lung, (Theranostics 2017; 7(6): 1543-1588; Pharmacological Research 103 (2016) 69-79).

The present inventors, with the aim of developing DF2755A as a drug, have undertaken investigations on the stability of the compound and have found that the known anhydrous DF2755A obtained in Example 3a of WO2010031835A2, is physically and chemically unstable. More particularly, as will be described in detail in Example 2 below, this form is not only highly hygroscopic - spontaneously absorbing water from the environment, thus becoming amorphous, partially melted, sticky and difficult to handle - but also converts, to a significant extent, into an undesired by-product.

A primary requisite for pharmaceutical products is that the active substance must have a stable crystalline morphology to ensure consistent processing parameters and quality, stability and reproducibility of the final drug product.

SUMMARY OF THE INVENTION

The inventors have found that the anhydrous solid form of DF2755A was not suitable for pharmaceutical use, especially for macro-scale applications, due to hygroscopicity and chemical instability.

The inventors have thus focused research activity aimed at obtaining a solid form of DF2755A which is physically and chemically stable, easy to handle, industrially advantageous and suitable for pharmaceutical applications.

After extensive studies, the inventors have surprisingly found that under very specific environmental conditions the unstable anhydrous DF2755A smoothly converted into a new stable crystalline monohydrate polymorph. Surprisingly, only the specific process conditions identified by the inventors, described in the present application, resulted in the stable monohydrate polymorph of the invention, while many other attempts to obtain a stable DF2755A form resulted in either the same known anhydrous DF2755A form, even when water was present in the crystallization medium, or non-stoichiometric unstable hydrates. This monohydrate DF2755A polymorph (herein named Form I) is physically and chemically stable, as shown by the analyses and the stability studies reported in the present Experimental section. Advantageously, this new crystalline form is not hygroscopic and does not undergo undesired chemical transformations.

Thus, a first aspect of the present invention provides a crystalline monohydrate sodium (2S)-2-(4-{[4-(trifluoromethyl)-1 ,3-thiazol-2-yl] amino} phenyl) propanoate characterized by an X ray diffraction pattern (XRPD) with relevant peaks at 7.9, 18.3, 19.8, 23.8 and 25.5° 2-theta ± 0.2 degrees 2-theta (hereinafter also referred to as "monohydrate DF2755A").

A further aspect of the present invention provides a process for the preparation of crystalline monohydrate sodium (2S)-2-(4-{[4-(trifluoromethyl)-1 ,3-thiazol-2-yl] amino} phenyl) propanoate comprising:

i) providing anhydrous sodium (2S)-2-(4-{[4-(trifluoromethyl)-1,3-thiazol-2-yl] amino} phenyl) propanoate; and
ii) exposing it to an atmosphere having a Relative Humidity (R.H.) higher than 60% and lower than 80% by weight, at a temperature from 25°C to 40 °C and at atmospheric pressure, preferably for a time from 12 to 72 hours, thus providing crystalline monohydrate sodium (2S)-2-(4-{[4-(trifluoromethyl)-1,3-thiazol-2-yl] amino} phenyl) propanoate.

A further aspect of the present invention provides a process suitable for large-scale preparation of anhydrous sodium (2S)-2-(4-{[4-(trifluoromethyl)-1,3-thiazol-2-yl] amino} phenyl) propanoate comprising:

- providing a solution of (2S)-2-(4-{[4-(trifluoromethyl)-1,3-thiazol-2-yl] amino} phenyl) propanoic acid in a solvent selected among ethyl acetate, isobutyl acetate, propyl acetate and admixtures thereof, in which the concentration of the acid in the solution is from 0.08 to 0.12 Kg/l;
- adding to the solution a sodium base in a molar ratio compared to the acid from 0.9:1 to 0.95:1, thus providing an admixture;
- adding to the admixture an anti-solvent selected among toluene, m-xylene, p-xylene and admixtures thereof, the anti-solvent being in a volumetric ratio compared to the solvent from 0.4:1 to 0.6:1, thus precipitating anhydrous sodium (2S)-2-(4-{[4-(trifluoromethyl)-1,3-thiazol-2-yl] amino} phenyl) propanoate.

A further aspect of the present invention provides a pharmaceutical composition comprising a therapeutically effective amount of crystalline monohydrate sodium (2S)-2-(4-{[4-(trifluoromethyl)-1 ,3-thiazol-2-yl] amino} phenyl) propanoate and at least a pharmaceutically acceptable excipient.

A further aspect of the present invention provides a crystalline monohydrate sodium (2S)-2-(4-{[4-(trifluoromethyl)-1,3-thiazol-2-yl] amino} phenyl) propanoate for use as a medicament.

A further aspect of the present invention provides a crystalline monohydrate sodium (2S)-2-(4-{[4-(trifluoromethyl)-1,3-thiazol-2-yl] amino} phenyl) propanoate for use in the prevention or treatment of an acute or chronic inflammatory-mediated disease, inflammatory and post-operative pain, interstitial cystitis (IC)/ bladder pain syndrome (BPS) and/or cancer.

A further aspect of the present invention provides a method for the prevention or treatment of acute or chronic inflammatory-mediated disease, inflammatory and post-operative pain, interstitial cystitis (IC)/ bladder pain syndrome (BPS) and/or cancer comprising administering to an individual in need thereof an effective amount of the crystalline monohydrate sodium (2S)-2-(4-{[4-(trifluoromethyl)-1,3-thiazol-2-yl] amino} phenyl) propanoate of the invention, alone or in combination with another pharmaceutically active ingredient.

DEFINITIONS

As used herein, the term "comprise," or variations thereof such as "comprises" or "comprising," are to be read to indicate the inclusion of any recited integer (e.g. a feature, element, characteristic, property, method/process step or

limitation) or group of integers (e.g. features, element, characteristics, properties, method/process steps or limitations) but not the exclusion of any other integer or group of integers. Thus, as used herein the term "comprising" is inclusive or open-ended and does not exclude additional, unrecited integers or method/process steps.

As used herein, the term "consisting of" is used to indicate the presence of the recited integer (e.g. a compound, a composition, a feature, element, characteristic, property, method/process step or limitation) or group of integers (e.g. compounds, compositions, features, element, characteristics, properties, method/process steps or limitations) alone. The phrase "consisting essentially of' is used herein to require the recited integer(s) as well as those which do not materially affect the character or function of the claimed invention. The term "substantially in accordance" with reference to XRPD diffraction patterns means that allowance is made for variability in peak positions and relative intensities of the peaks. The ability to ascertain substantial identities of X-ray diffraction patterns is within the purview of one of ordinary skill in the art. For example, a typical precision of the 2-Theta values is in the range of ± 0.2° 2-Theta. Thus, a diffraction peak that usually appears at 14.9° 2-Theta can appear between 14.7° and 15.1 °2-Theta on most X-ray diffractometers under standard conditions. XRPD measurements are typically performed at RT, for example at a temperature of 20°C, and preferably also at a relative humidity of 40%.

For the purpose of the present invention, the term "pharmaceutically acceptable excipient" refers to a substance devoid of any pharmacological effect of its own and which does not produce adverse reactions when administered to a mammal, preferably a human.

For the purpose of the present invention, the term "room temperature" (RT) means a temperature range of 18 to 25 °C.

For the purpose of the present invention, the term "anti-solvent" means a solvent in which a compound is insoluble or poorly soluble.

The terms "approximately", "around" and "about" herein refer to the range of the experimental error, which may occur in a measurement.

## BRIEF DESCRIPTION OF THE FIGURES

Figure 1 shows the $^{1}$H-NMR spectrum (solvent: $D_2O$) of the anhydrous DF2755A prepared according to Example 3a of WO2010031835A2 measured at time 0;

Figure 2 shows the XRPD pattern of the anhydrous DF2755A prepared according to Example 2A measured at time 0 (y-axis: counts);

Figure 3 shows the $^{1}$H-NMR spectrum (solvent $D_2O$) of the anhydrous DF2755A prepared according to Example 2A after exposure to uncontrolled ambient humidity for 24 hours at a temperature of about 25°C (the signals of the hydroxylated by-product are marked with Xs);

Figure 4 shows the XRPD pattern of the anhydrous DF2755A prepared according to Example 2A after exposure to humidity (%RH: 50%) for 24 hours at a temperature of about 25°C (y-axis: counts);

Figure 5 shows the $^{1}$H-NMR spectrum (solvent $D_2O$) of monohydrate DF2755A prepared according to Example 3, measured after 24 hours in the climatic room at 65% R.H. and 30 °C;

Figure 6 shows the XRPD pattern of monohydrate DF2755A Form I prepared according to Example 3, measured at time 0 and after 24 hours in the climatic room at 65% R.H. and 30 °C (y-axis: counts);

Figure 7 shows the DSC analysis of monohydrate DF2755A kept at 30°C/65% R.H or 40°C/75% R.H., measured at time 0 and after 1, 3, 6, 9, 12 and 18 months.

Figure 8 shows the HPLC chromatogram of monohydrate DF2755A.

## DETAILED DESCRIPTION OF THE INVENTION

A first aspect of the present invention provides a crystalline monohydrate sodium (2S)-2-(4-{[4-(trifluoromethyl)-1,3-thiazol-2-yl] amino} phenyl) propanoate characterized by an X ray diffraction pattern (XRPD) with relevant peaks at 7.9, 18.3, 19.8, 23.8 and 25.5° 2-theta ± 0.2 degrees 2-theta (i.e. with a margin of error on the value indicated for each peak of ± 0.2 degrees 2-theta).

For short, this compound is herein named "monohydrate DF2755A".

The monohydrate DF2755A is characterized by the $^{1}$H-NMR spectrum of Figure 5, the XRPD of Figure 6 and the DSCs of Figure 7.

One of ordinary skill in the art will appreciate that an XRPD pattern may be obtained with a measurement error that is dependent upon the measurement conditions employed. It will be appreciated that the crystalline form described herein is not limited to the crystalline form that yield X-ray diffraction patterns completely identical to the X-ray diffraction patterns depicted in the accompanying Figures. Rather, crystalline forms of DF2755A that provide X-ray diffraction

patterns substantially in accordance (as hereinbefore defined) with those shown in Figure 6 fall within the scope of the present invention.

The crystalline form of the monohydrate DF2755A obtained in the present invention, characterized by an X ray diffraction pattern (XRPD) with relevant peaks at 7.9, 18.3, 19.8, 23.8 and 25.5°2-theta ± 0.2 degrees 2-theta, is herein named Form I.

The present crystalline form of the monohydrate DF2755A is preferably further characterized by XRPD relevant peaks at 9.3, 15.5, 22.1, 22.3, 23.0 and 24.6°2theta ± 0.2 degrees 2-theta.

Other polymorphs of the present monohydrate DF2755A are also within the scope of the invention.

All the most relevant XRPD peaks of the monohydrate DF2755A are shown in the following Table 1:

Table 1

| Pos. [°2Th.] | Rel. Int. [%] |
|---|---|
| 7.9 | 99.9 |
| 9.3 | 8.1 |
| 15.5 | 7.9 |
| 17.5 | 4.1 |
| 18.3 | 16.3 |
| 19.8 | 16.8 |
| 22.1 | 8.2 |
| 22.3 | 8.5 |
| 23.0 | 8.7 |
| 23.8 | 16.8 |
| 24.6 | 9.6 |
| 25.5 | 16.9 |
| 27.5 | 5.1 |
| 28.1 | 5.1 |
| 29.1 | 5.1 |

The monohydrate DF2755A of the present invention preferably has a water content from 5.0% to 6.3% by weight, more preferably from 5.0% to 5.5% by weight, even more preferably from 5.0% to 5.3% by weight, measured by Karl Fischer method.

The monohydrate DF2755A of the invention appears as a white powder.

This powder is characterized by good rheological properties and is particularly suitable for pharmaceutical applications. As known in the art, technological properties of powders (PSD, bulk density, flowability, surface area, etc.) as well as their use in pharmaceutics strictly depend on particle characteristics.

The powder of monohydrate DF2755A obtained by the present process is non-sticky and shows improved appearance compared to that of the anhydrous DF2755A. Furthermore, the powder properties and morphology of the present monohydrate are particularly advantageous for drug formulation.

Preferably the monohydrate DF2755A powder of the present invention is characterized by the following particle size distribution intervals: D(0.1) = 2.0 - 2.5 μm, D(0.5) = 6.5 - 8.5 μm, D(0.9) = 20.0 - 30.0 μm, as measured by Malvern Mastersizer 3000 using Aero S accessory.

The bulk density of a powder is the ratio of the mass of an untapped powder sample to its volume. It depends on both the density of powder particles and the spatial arrangement of the particles in the powder bed.

Preferably the monohydrate DF2755A powder of the present invention is characterized by a bulk density higher than 0.15 g/ml, more preferably higher than 0.16 g/ml, higher than 0.17 g/ml, even more preferably from 0.17 to 0.20 g/ml measured according to Ph. Eur. 2.9.34.

Preferably the monohydrate DF2755A powder of the present invention is characterized by a tapped density from 0.19 g/ml to 0.28 g/ml, preferably around 0.25 g/ml measured according to Ph. Eur. 2.9.34.

Preferably the monohydrate DF2755A powder of the present invention is characterized by a compressibility index from 23 to 33.

Preferably the monohydrate DF2755A powder of the present invention is characterized by a Hausner ratio lower than 1.50, more preferably lower than 1.45, typically from 1.30 to 1.45.

As shown in the Experimental section, the monohydrate DF2755A powder of the present invention is not hygroscopic (see the unchanged water content for at least 12 months under accelerated stability conditions - Table 7).

Furthermore, the monohydrate DF2755A according to the invention is chemically and optically stable (see the unchanged impurities content and enantiomeric purity in Table 7 in the Experimental Section). In particular, the by-product of hydroxylation at position 4 of the thiazole ring ((2S)-2-(4-{[4-hydroxy-4-(trifluoromethyl)-4,5-dihydro-1,3-thiazol-2-yl]amino}phenyl)propanoic acid (herein named DFL23803) that originates in significant amount when exposing the anhydrous DF2755A at ambient uncontrolled humidity at a temperature of about 25 °C, was not detected. Also the undesired enantiomer (2R)-2-(4-{[4-(trifluoromethyl)-1,3-thiazol-2-yl]amino}phenyl)propanoic acid (herein named DF2703Y) is well below product specifications (see Table 7).

Therefore, in accordance with the present invention there is provided a composition comprising, consisting essentially of or consisting of DF2755A, preferably a composition comprising, consisting essentially of or consisting of the monohydrate DF2755A, as described herein.

Preferably the total impurities content in the present composition is lower than 0.5%, more preferably lower than 0.1%, even more preferably lower than 0.05%, measured by HPLC as described in the present experimental section (see for instance the data of Table 7).

Preferably the present composition comprises or consists essentially of DF2755A, preferably the monohydrate DF2755A as described above, with a content of the structurally related impurity (2S)-2-(4-{[4-hydroxy-4-(trifluoromethyl)-4,5-dihydro-1,3-thiazol-2-yl]amino}phenyl)propanoic acid (DFL23803) lower than 0.5%, preferably lower than 0.2%, more preferably lower than 0.1%, determined by HPLC method according to the description (see for instance the data of Table 5 and Table 7).

In conclusion, the monohydrate DF2755A of the invention is advantageous over the known anhydrous DF2755A and particularly suitable for large-scale pharmaceuticals applications. A further aspect of the present invention provides a process for the preparation of crystalline monohydrate sodium (2S)-2-(4-{[4-(trifluoromethyl)-1,3-thiazol-2-yl] amino} phenyl) propanoate comprising:

i) providing anhydrous sodium (2S)-2-(4-{[4-(trifluoromethyl)-1,3-thiazol-2-yl] amino} phenyl) propanoate; and

ii) exposing it to an atmosphere having a Relative Humidity (R.H.) higher than 60% and lower than 80% by weight, at a temperature from 25°C to 40 °C and at atmospheric pressure, preferably for a time from 12 to 72 hours, thus providing crystalline monohydrate sodium (2S)-2-(4-{[4-(trifluoromethyl)-1,3-thiazol-2-yl] amino} phenyl) propanoate.

Regarding step i) of the present process, the starting anhydrous sodium (2S)-2-(4-{[4-(trifluoromethyl)-1,3-thiazol-2-yl] amino} phenyl) propanoate (hereinafter also referred to as "anhydrous DF2755A") is preferably prepared according to the process of the invention described in the following.

Regarding step ii) of the present process, the atmosphere to which the anhydrous DF2755A is exposed can be air, one or more inert gasses or their admixtures, preferably the atmosphere is nitrogen.

Regarding the R.H. of the atmosphere, the inventors observed that for values up to 60% of R.H. there was little or no conversion to the monohydrate while for R.H. values of 80% or higher the product becomes deliquescent.

Preferably, the atmosphere R.H. is from 65% to 75% by weight.

Preferably, the atmosphere temperature is from 25° to 30°C.

Preferably, the time of exposure is from 24 to 72 hours.

A preferred process for the preparation of crystalline monohydrate sodium (2S)-2-(4-{[4-(trifluoromethyl)-1,3-thiazol-2-yl] amino} phenyl) propanoate comprises:

i) providing anhydrous sodium (2S)-2-(4-{[4-(trifluoromethyl)-1,3-thiazol-2-yl] amino} phenyl) propanoate, preferably prepared according to the process of the invention described below; and

ii) exposing it to an atmosphere having a Relative Humidity (R.H.) from 65% to 75% by weight, at a temperature from 25°C to 30°C and at atmospheric pressure, preferably for a time from 12 to 72 hours, thus providing crystalline monohydrate sodium (2S)-2-(4-{[4-(trifluoromethyl)-1,3-thiazol-2-yl] amino} phenyl) propanoate.

Typically, the anhydrous DF2755A is spread onto a tray and left in a climate room set with the desired R.H. and T for the time needed to complete the conversion. The conversion can be monitored by Karl Fischer analysis.

A further aspect of the present invention provides a process suitable for large-scale preparation of anhydrous sodium (2S)-2-(4-{[4-(trifluoromethyl)-1,3-thiazol-2-yl] amino} phenyl) propanoate comprising:

- providing a solution of (2S)-2-(4-{[4-(trifluoromethyl)-1,3-thiazol-2-yl] amino} phenyl) propanoic acid (DF2755Y) in

a solvent selected among ethyl acetate, isobutyl acetate, propyl acetate and admixtures thereof, in which the concentration of the acid in the solution is from 0.08 to 0.12 Kg/l,

- adding to the solution a sodium base in a molar ratio compared to the acid from 0.9:1 to 0.95:1, thus providing an admixture,
- adding to the admixture an anti-solvent selected among toluene, m-xylene, p-xylene and admixtures thereof, the anti-solvent being in a volumetric ratio compared to the solvent from 0.4:1 to 0.6:1, thus precipitating anhydrous sodium (2S)-2-(4-{[4-(trifluoromethyl)-1,3-thiazol-2-yl] amino} phenyl) propanoate.

Preferably, the concentration of the acid DF2755Y in the solution is from 0.09 to 0.11 Kg/l, more preferably around 0.10 Kg/l

Preferably, the solvent is selected among isobutyl acetate, propyl acetate and admixtures thereof, more preferably the solvent is isobutyl acetate.

Preferably, the sodium base is selected among sodium hydroxide or sodium methoxide, more preferably is sodium hydroxide. The sodium base can be added as such or, preferably, dissolved in a suitable solvent, preferably dissolved in water or in aqueous admixtures with polar solvents such as, for instance, ethanol or methanol. In a preferred embodiment, the sodium base is aqueous sodium hydroxide.

Preferably, the sodium base is added substoichiometric with respect to the acid in order to prevent racemization of the chiral carbon.

According to the present process, the precipitation of the anhydrous DF2755A from the solution is carried out by addition of anti-solvent preferably selected among toluene, p-xylene and admixtures thereof, more preferably the anti-solvent is toluene.

Preferably, the volumetric ratio of the anti-solvent or of the admixtures thereof compared to the solvent is around 0.5:1.

The precipitation is typically carried out under stirring, preferably by cooling at temperatures lower than 20°C, more preferably lower than 10°C, even more preferably at about 5°C.

A preferred process suitable for large-scale preparation of anhydrous sodium (2S)-2-(4-{[4-(trifluoromethyl)-1,3-thiazol-2-yl] amino} phenyl) propanoate comprises:

- providing a solution of (2S)-2-(4-{[4-(trifluoromethyl)-1,3-thiazol-2-yl] amino} phenyl) propanoic acid (DF2755Y) in isobutyl acetate, in which the concentration of the acid in the solution is from 0.09 to 0.11 Kg/l, preferably around 0.10 Kg/l,
- adding to the solution sodium hydroxide in a molar ratio compared to the acid from 0.9:1 to 0.95:1, thus providing an admixture,
- adding to the admixture toluene in a volumetric ratio compared to the solvent of about 0.5:1, thus precipitating anhydrous sodium (2S)-2-(4-{[4-(trifluoromethyl)-1,3-thiazol-2-yl] amino} phenyl) propanoate.

Advantageously, the present process for the preparation of the anhydrous DF2755A by anti-solvent precipitation, is industrially feasible and avoids the troublesome and expensive lyophilization procedure shown in the prior art. The present process provides anhydrous sodium (2S)-2-(4-{[4-(trifluoromethyl)-1,3-thiazol-2-yl] amino} phenyl) propanoate with enhanced chemical and enantiomeric purity in comparison with the lyophilization process of prior art.

A further aspect of the present invention provides a pharmaceutical composition comprising a therapeutically effective amount of crystalline monohydrate sodium (2S)-2-(4-{[4-(trifluoromethyl)-1 ,3-thiazol-2-yl] amino} phenyl) propanoate and at least a pharmaceutically acceptable excipient.

The choice of the excipients will to a large extent depend on factors such as the particular mode of administration, the effect on solubility and stability, and the nature of the dosage form.

Pharmaceutical compositions according to the present invention may be in any form suitable for the application to humans and/or animals, preferably humans including infants, children and adults and can be produced by standard procedures known to those skilled in the art.

In one embodiment, the pharmaceutical composition of the present invention is an oral solid composition, such as for instance a capsule, pellet, tablet, cachet, chewable dosage forms, powder, lozenge, granules, oral soluble granulate, suspension, emulsion, spray, or as dry powdered form to be reconstituted with a liquid medium.

The pharmaceutical composition can additionally contain one or more pharmaceutically acceptable excipients, such as fillers, binders, glidants, disintegrants, flow regulating agents and release agents.

Suitable excipients are for example disclosed in "Handbook of Pharmaceutical Excipients", 3rd Edition, published by A.H. Kibbe, American Pharmaceutical Association, Washington, USA, and Pharmaceutical Press, London.

Suitable fillers are for example lactose, mannitol, xylitol, dextrose, sucrose, sorbitol, microcrystalline cellulose, starch, dibasic calcium phosphate dihydrate and calcium hydrogen phosphate.

Fillers can be present in an amount for instance of 0 - 80% by weight, preferably in an amount of 10 - 60% by

weight of the total weight of the composition.

Suitable binders are for example polyvinylpyrrolidone, microcrystalline cellulose hydroxypropyl cellulose, hydroxypropyl methylcellulose, methylcellulose, hydroxyethyl cellulose, sugars, dextran, cornstarch, gelatin, polyethylene glycol, natural and synthetic gums, pregelatinised starch.

Binders can be present in an amount of 0 - 80% by weight, preferably in an amount of 10 - 60% by weight of the total weight of the composition.

Binders are generally used to impart cohesive qualities to a tablet formulation.

Suitable glidants are for example alkaline earth metal salts of fatty acids, like stearic acid such as magnesium stearate, calcium stearate, zinc stearate, sodium stearyl fumarate, and mixtures of magnesium stearate with sodium lauryl sulphate.

The glidant can be present for example in an amount of 0 - 2% by weight, preferably in an amount of 0.5 - 1.5% by weight of the total weight of the composition.

Suitable disintegrants are for example crosscarmellose sodium, sodium carboxymethyl starch, crosslinked polyvinylpyrrolidone (crosspovidone), sodium carboxymethylglycolate, sodium starch glycolate, sodium carboxymethyl cellulose, calcium carboxymethyl cellulose, polyvinylpyrrolidone, methyl cellulose, microcrystalline cellulose, lower alkyl-substituted hydroxypropyl cellulose, starch, pregelatinised starch, sodium alginate and sodium bicarbonate.

The disintegrant can be present in an amount of 0 - 20% by weight, preferably in an amount of 1 - 15% by weight of the total weight of the composition.

A suitable flow-regulating agent is for example colloidal silica. The flow regulating agent can be present in an amount of 0 - 8% by weight, preferably in an amount of 0.1 - 3% by weight of the total weight of this composition.

A suitable release agent is for example talcum. The release agent can be present in an amount of 0 - 5% by weight, preferably in an amount of 0.5 - 3% by weight of the total weight of the composition.

The solid composition may be coated, preferably film coated.

Suitable coating agents are for example cellulose derivatives, poly(meth)acrylate, polyvinyl pyrrolidone, polyvinyl acetate phthalate, and/or shellac or natural rubbers such as carrageenan.

There are many situations in which it will be advantageous or even necessary to deliver the monohydrate DF2755A of the present invention as a solid, for instance by installing a solid implant composition into suitable body tissues or cavities.

The implant may comprise a matrix of bio-compatible and bioerodible materials in which particles of the monohydrate DF2755A of the present invention are dispersed, or in which, possibly, globules or isolated cells of a liquid mixture of the present monohydrate DF2755A are entrapped. Desirably, the matrix will be broken down and completely absorbed by the body. The composition of the matrix is also preferably selected to provide controlled-, sustained-, and/or delayed release of the monohydrate DF2755A of the present invention over extended periods.

Alternatively, the monohydrate DF2755A of the invention may be formulated as a solid, semi-solid, or thixotropic liquid for administration as an implanted depot providing modified release of the active compound.

The present composition can be administered topically to the skin or mucosa, that is dermally, epidermally, subepidermally or transdermally.

The present composition can be administered sublingually or via a suppository.

Typical formulations for this purpose include pour-on, spot-on, dip, spray, mousse, shampoo, powder formulation, gels, hydrogels, lotions, creams, ointments, dusting powders, dressings, foams, films, skin patches, wafers, implants, depots, sponges, fibres, bandages, microemulsions, orosoluble granulates. Liposomes may also be used.

The pharmaceutical composition of the present invention may be a solution or a suspension, for oral or parenteral administration, to be administered for example by intramuscular, intraperitoneal, or intravenous injection

The pharmaceutical composition of the present invention may be a solid composition for the extemporaneous preparation of a solution for oral or parenteral administration, to be administered for example by intramuscular, intraperitoneal, or intravenous injection.

The pharmaceutical composition of the present invention can be prepared by methods well known to a person skilled in the art.

The composition of the invention may be of immediate-, delayed-, modified-, sustained-, pulsed- or controlled-release type.

According to a further embodiment, the pharmaceutical composition of the invention may comprise the monohydrate DF2755A of the invention and at least another pharmaceutically active ingredient.

The other pharmaceutically active ingredient will be determined by the circumstances under which the therapeutic agent of the present invention is administered.

The other pharmaceutically active ingredient is for instance selected among analgesic and/or anti-inflammatory drugs or among anti-cancer drugs.

A further aspect of the present invention provides crystalline monohydrate sodium (2S)-2-(4-{[4-(trifluoromethyl)-1,3-thiazol-2-yl] amino} phenyl) propanoate as described above for use as a medicament.

The medical use can be curative, prophylactic or palliative.

A further aspect of the present invention provides crystalline monohydrate sodium (2S)-2-(4-{[4-(trifluoromethyl)-1,3-thiazol-2-yl] amino} phenyl) propanoate as described above for use in the prevention or treatment of an acute or chronic inflammatory-mediated disease, inflammatory and post-operative pain, interstitial cystitis (IC)/ bladder pain syndrome (BPS), and/or cancer.

Preferably, said inflammatory-mediated disease is selected from bullous pemphigoid, cystic fibrosis, chronic obstructive pulmonary disorder, asthma, psoriasis, rheumatoid arthritis and inflammatory bowel disease.

Preferably said cancer is selected from lung, colon, prostate, pancreatic, breast and ovarian cancer and melanoma.

The daily dosage for humans and animals may vary depending on factors that have their basis in the respective species or other factors, such as age, sex, weight or degree of illness and so forth.

A further aspect of the present invention provides a method for the prevention or treatment of pain, inflammation and/or cancer comprising administering to an individual in need thereof a therapeutically effective amount of the monohydrate DF2755A of the invention, alone or in combination with another pharmaceutically active ingredient.

According to the present invention, the term "individual" refers to a human or an animal being, preferably to a human being.

The same preferences expressed above for the monohydrate DF2755A medical uses apply to the present method as well.

## EXPERIMENTAL SECTION

In the following some non-limitative examples, according to the invention or comparative, are provided for illustrative purposes.

### Analytical and test methods

#### HPLC assay:

Apparatus: Agilent HP 1100, HP 1200 or equivalent HPLC system with UV detector
Detector: wavelength: 220 nm
Column: BDS Hypersyl C18, 250 × 4.6mm, 5μm (Thermo) or equivalent
Column temperature: 35°C
Mobile phase: (A) Buffer solution $KH_2PO_4$ 0.025M (pH 3.0). Procedure: dissolve 3.4 g of potassium dihydrogen phosphate in water and dilute to 1000 ml with water; adjust to pH 3.0 with dilute phosphoric acid or with potassium hydroxide solutions. (B) acetonitrile.
Flow: 1.3 ml/minute
Injection Volume: 10 μl
Time between injections: 26 min
Diluent: $H_2O/CH_3CN$ (40/60)
Relative retention time: DF 2755 Rt 11.8 min
Gradient: Table 2

Table 2

| Time (min) | Mobile phase A (%) | Mobile phase B (%) |
|---|---|---|
| 0 | 60 | 40 |
| 10 | 60 | 40 |
| 19 | 40 | 60 |
| 20 | 60 | 40 |
| 26 | 60 | 40 |

#### HPLC enantiomeric purity

Chromatographic conditions
Apparatus: Agilent HP 1100, HP 1200 or equivalent HPLC system with UV detector
Detector: wavelength: 294 nm

Column: Chiralpak AS-RH, 150 × 4.6 mm 55μm (Daicel) or equivalent
Column temperature: 35 °C
Mobile phase: (A) Buffer solution $KH_2PO_4$ 0.025M (pH 3.0) Procedure: dissolve 3.4 g of potassium dihydrogen phosphate in water and dilute to 1000 ml with water; adjust to pH 3.0 with dilute phosphoric acid or with potassium hydroxide solutions; (B) acetonitrile
Flow: 0.7 ml/minute
Injection volume: 10 μl
Time between injections: 45 min
Diluent: $H_2O/CH_3CN$ (40/60)
Eluent: 70% Mobile phase A, 30% Mobile phase B
$^1$H-NMR: $^1$H-Nuclear magnetic resonance (NMR) spectra were recorded in the indicated solvent with tetramethylsilane (TMS) as internal standard on a Bruker Avance3 400 MHz instrument.
XRPD: The XRPD analysis was carried out at RT with the following instrument and under the conditions reported in Table 3 below:

Table 3

| | |
|---|---|
| Instrument type: | Rigaku MiniFlex600 |
| Application SW: | Miniflex Guidance |
| Measurement Details | |
| Measurement type: | Single scan |
| Sample mode: | Reflection |
| Scan | |
| Scan range: | 3.000 - 40.000 °(2θ) |
| Step size: | 0.01 °(2θ) |
| Speed: | 10.0 °/min (2θ) |
| Scan mode: | Continuous |
| Used wavelength | |
| Intended wavelength type: | Kα1 |
| Kα1: | 1.540598 Å |
| Kα2: | 1.544426 Å |
| Kα2/Kα1 intensity ratio: | 0.50 |
| Kα: | 1.541874 Å |
| Kα: | 1.392250 Å |
| Instrument Details | |
| X-Ray Generator | |
| Tube output voltage: | 40 kV |
| Tube output: | 15 mA |
| High-voltage generation method: | High-frequency Cockcroft-Walton method |
| Stability: | Within ±0.05% for both the tube voltage and tube current, with reference to ±10% of input power variation. |
| X-ray tube | |
| Name: | Toshiba Analix type A-26L |
| Anode material: | Cu |
| Maximus output: | 0.60 kW |
| Focus size: | 1 x 10 mm |

(continued)

| | |
|---|---|
| Kβ Filter | |
| Name: | Ni-filter |
| Thickness (mm): | 0.015 |
| Material: | Ni |
| Goniometer (Angle measuring device) | |
| Type: | Vertical θ/2θ |
| Goniometer radius: | 150 mm |
| Scanning axis: | θ/2θ linked |
| 2θ scanning range: | +2 °to +140 ° |
| θ/2θ axis minimum step angle: | 0.005 °(2θ) |
| Position speed: | 500 °/min (2θ) |
| Scanning speed: | 0.01 to 100 °/min |
| Datum angle: | 2θ = 10 ° |
| X-ray take-off angle: | 6 ° (fixed) |
| Slit | |
| DS: | 1.25 ° |
| IHS: | 10.0 mm |
| SS: | none (open) |
| RS: | none (open) |
| Incident side Soller slit: | 2.5 ° |
| Receiving side Soller slit: | 2.5 ° |
| Detector | |
| Name: | D/teX Ultra High-speed 1D Detector |
| Window material: | Be |
| Effective window size: | 13 mm (H) x 20 mm (W) |
| Dimensions: | 80 mm (L) |

Water content by Karl-Fischer:

Instrument: Mettler DL38, V30 or equivalent
Electrode: Double platinum pin electrode DM 143-SC for Karl Fischer titration.
Titrant: Karl Fischer reagent 5 mg $H_2O$ /ml
Solvent: 30 ml of methanol previously neutralized with Karl Fischer reagent.
Procedure: about 0.2 g of the product, accurately weighed, were dissolved in the solvent and titrated with the Karl Fischer reagent.

$$\text{Formula: } \%\, H_2O = (V \times e) / (10 \times w)$$

where
V = ml of the Karl Fischer reagent used in the titration
e = equivalent of the Karl Fischer reagent (mg $H_2O$ /ml)

w = weight of the sample in g

The analysis was carried out on three samples and the results averaged. The limit had to be not more than 10.0 %.

Particle Size Distribution was measured by Malvern Mastersizer 3000 using Aero S accessory.

Tapped and Bulk Density were determined with a density tester ERWEKA SMV 102 (Heusenstamm, Germany) according to European Pharmacopeia (Ph. Eur. 2.9.34).

Compressibility Index was calculated according to the formula:

$$C.I.= 100 \times (\text{tapped density - bulk density})/\text{tapped density}.$$

Hausner ratio was calculated according to the formula:

$$H.r.= \text{tapped density / bulk density}$$

Example 1: preparation of (2S)-2-(4-{[4-(trifluoromethyl)-1,3-thiazol-2-yl] amino} phenyl) propanoic acid (DF2755Y)

The titled compound was prepared as described under Example 3 of WO2010031835A2.

Example 2: preparation of anhydrous sodium (2S)-2-(4-{[4-(trifluoromethyl)-1,3-thiazol-2-yl] amino} phenyl) propanoate (anhydrous DF2755A) (lab scale)

The titled compound was prepared starting from the free acid DF2755Y of Example 1 according to the procedure described under Example 3a of WO2010031835A2.

Example 2A (invention): preparation of anhydrous sodium (2S)-2-(4-{[4-(trifluoromethyl)-1,3-thiazol-2-yl] amino} phenyl) propanoate (anhydrous DF2755A) (pilot scale)

The acid DF2755Y of Example 1 (7.9 kg) was charged in a 200 litre glass chemical reactor, then 63 litres of isobutyl acetate were added at room temperature (r.t.) under stirring. Decolorizing carbon (0.4 kg) was added to the dark solution and left for 20 minutes at r.t. The solution was filtered on a FAP5 filter obtaining a perfectly clear light-yellow solution. The solution was charged in a 500 litre glass chemical reactor and 16 litres of isobutyl acetate were added at r.t. under stirring. Sodium hydroxide 50% in aqueous solution (1.3 litres) were added and the resulting solution mixed for 1 hour at 50°C. The reactor was cooled at 20°C, and then toluene (40 litres) was added. After 1 hour under stirring the product started to precipitate and the admixture was further stirred for 12 hours at 5°C. The slurry was then centrifuged at 1000 rpm for 30 minutes. The isolated product was dried under vacuum at 40°C for about 24 hours to afford crystalline anhydrous DF2755A as a white solid (8.1 kg, 96% yield) with m.p. of about 150°C.

The anhydrous DF2755A so obtained was characterized by $^1$H-NMR (at T0, $^1$H-NMR spectrum in accordance with the spectrum of the anhydrous DF2755A prepared according to Example 3a of WO2010031835, shown in Figure 1) and by XRPD analysis (Figure 2). The relevant XRPD peaks of the anhydrous DF2755A had the positions and relative intensities shown in the following Table 4:

Table 4

| Pos. [°2Th.] | Rel. Int. [%] |
|---|---|
| 8.1 | 100 |
| 9.5 | 67.7 |
| 15.7 | 29.3 |
| 18 | 7.1 |
| 18.8 | 30.1 |
| 19.2 | 57.1 |
| 19.8 | 8.1 |
| 22.2 | 15.1 |

(continued)

| Pos. [°2Th.] | Rel. Int. [%] |
|---|---|
| 22.5 | 21.1 |
| 23.5 | 14.7 |
| 24.1 | 100 |
| 24.6 | 42.5 |
| 25.5 | 10.1 |
| 27.7 | 11.1 |
| 28.5 | 14.9 |
| 29.5 | 13.1 |

Stability test

A sample of anhydrous DF2755A prepared according to Example 2A was placed in a climatic chamber and exposed to 50% R.H. at about 25°C for 24 hours thus providing a sticky hydrated solid with a water content of 17% measured by Karl Fischer analysis. This solid showed the XRPD spectrum of Figure 4.

$^1$H-NMR analysis showed that the hydrated solid comprised another compound, namely the by-product of hydroxylation at position 4 of the thiazole ring of DF2755A in significant amount (about 50% mol/mol) (see in Figure 3 the relevant signals marked by Xs).

In conclusion, anhydrous DF2755A under the test conditions was both physically and chemically unstable.

Example 3 (invention): preparation of monohydrate sodium (2S)-2-(4-{[4-(trifluoromethyl)-1,3-thiazol-2-yl] amino} phenyl) propanoate Form I (monohydrate DF2755A Form I) by controlled hydration

Anhydrous DF2755A prepared according to Example 2A (0.040 kg) was spread on a tray and placed in a climatic chamber at 65% R.H., at a temperature of 30 °C for 48 hours thus providing monohydrate DF2755A Form I as a white solid (0.039 kg, 93% yield).

The final product was analyzed by HPLC providing the chromatogram of Figure 8 and the results shown in Table 5 below:

Table 5

| Name | Retention Time | % Area | RRT |
|---|---|---|---|
| DFL23803 | 4.708 | 0.02 | 0.387 |
| DF2755A | 12.175 | 99.47 | 1.000 |
| Single unknown impurity | 14.952 | 0.01 | 1.228 |
| Single unknown impurity | 24.470 | 0.01 | 2.010 |

As can be seen, the final monohydrate DF2755A had a very high purity and contained the by-product DFL23803 in negligible amount (0.02%).

The product was also analyzed by Karl Fisher method (water content: 5.27% by weight), by $^1$H-NMR (after 24 hours following removal from the climatic chamber, Figure 5) and by XRPD (at time 0 and after 24 hours following removal from the climatic chamber, Figure 6). The relevant XRPD peaks of the monohydrate DF2755A, at T0 and after 24 hours, had the positions and relative intensities shown in the following Table 6:

Table 6

| T0 | | T24 | |
|---|---|---|---|
| Pos. [°2Th.] | Rel. Int. [%] | Pos. [°2Th.] | Rel. Int. [%] |
| 7.9 | 100 | 7.9 | 100 |

(continued)

| T0 | | T24 | |
|---|---|---|---|
| Pos. [°2Th.] | Rel. Int. [%] | Pos. [°2Th.] | Rel. Int. [%] |
| 9.3 | 8.1 | 9.2 | 34.1 |
| 15.5 | 7.9 | 15.4 | 25.8 |
| 17.5 | 4.1 | 17.5 | 43.6 |
| 18.3 | 16.3 | 18.3 | 99.7 |
| 19.8 | 16.8 | 19.8 | 24.9 |
| 22.1 | 8.2 | 22.1 | 47.1 |
| 22.3 | 8.5 | 22.2 | 49.8 |
| 23.0 | 8.7 | 23.0 | 36.5 |
| 23.8 | 16.8 | 23.8 | 57.4 |
| 24.6 | 9.6 | 24.6 | 50.2 |
| 25.5 | 16.9 | 25.4 | 35.1 |
| 27.5 | 5.1 | 27.5 | 26.1 |
| 28.1 | 5.1 | 28.1 | 26.1 |
| 29.1 | 5.1 | 29.0 | 26.1 |

These analyses confirmed the structure, purity, crystallinity and stability of the monohydrate DF2755A Form I.

Stability test

The monohydrate DF2755A was then subjected to stability tests under the following conditions:

A) at 30°C and 65% R.H. (up to 18 months) and
B) at 40°C and 75% R.H. (accelerated stability up to 12 months).

The samples were analysed for appearance, water content, HPLC purity, HPLC assay, HPLC enantiomeric purity and DSC thermal behaviour. The outcome of the analyses is shown in Table 7 below:

Table 7: stability data under accelerated conditions B

| | | Time (months) | | | | |
|---|---|---|---|---|---|---|
| Test | Limits | 0 | 1 | 3 | 6 | 12 |
| Appearance | White to ivory powder | Compl. | Compl. | Compl. | Compl. | Compl. |
| Water content (K.F %) | < 10.0 | 5.27 | 5.27 | 5.12 | 4.95 | 5.17 |
| Total impurities content % (HPLC) | < 1.0% | 0.03 | 0.03 | 0.03 | 0.03 | 0.02 |
| DFL23803 (HPLC) | ≤0.11% | ≤0.11% | ≤0.11% | ≤0.11% | ≤0.11% | ≤0.11% |
| Enantiomeric purity % (HPLC) | | | | | | |
| DF2703Y | < 3.0 | 1.0 | 1.0 | 1.0 | 1.0 | 1.0 |
| Assay % (HPLC) | | | | | | |

(continued)

| Assay % (HPLC) | | | | | | |
|---|---|---|---|---|---|---|
| | > 95 | 97.40 | 96.93 | 97.67 | 96.94 | 100.3 |
| Compl: compliant; n.d.: not detected;<br>DF2703Y: (2R)-2-(4-{[4-(trifluoromethyl)-1,3-thiazol-2-yl]amino}phenyl)propanoic acid;<br>DFL23803: (2S)-2-(4-{[4-hydroxy-4-(trifluoromethyl)-4,5-dihydro-1,3-thiazol-2-yl]amino} phenyl)propanoic acid, and in Figure 7, where the monohydrate DF2755A showed substantially the same DSC profile over time under both stability test conditions A) and B). | | | | | | |

From these data it appeared that monohydrate DF2755A was completely stable at least for 18 months under conditions A and at least for 12 months under accelerated conditions B (see Figure 7 and Table 7), in particular formation in significant amount of the hydroxylated by-product DFL23803 and of the other enantiomer (2R) DF2703Y was not observed. All the results of analytical tests remained within the specification limits for the entire duration of the study, and there was no evidence of formation of degradation compounds.

Example 3A (invention): preparation of monohydrate sodium (2S)-2-(4-{[4-(trifluoromethyl)-1,3-thiazol-2-yl] amino} phenyl) propanoate Form I (monohydrate DF2755A Form I) by controlled hydration

Anhydrous DF2755A, prepared according to Example 2A (8.1 kg), was spread onto a tray and placed in a climatic chamber at 75% R.H., at a temperature of 25 °C for 72 hours thus providing monohydrate DF2755A Form I as a white solid (8.5 kg, 100% yield). The product had a water content of 5.27% by weight by Karl Fisher method and an XRPD compliant with the monohydrate DF2755A Form I.

The monohydrate DF2755A Form I powder showed the following rheological properties:

- Particle size distribution: D(0.1) = 2.09 μm, D(0.5) = 7.86 μm, D(0.9) = 25.42 μm;
- Tapped density: 0.256 g/ml;
- Bulk density: 0.178 g/ml;
- Compressibility index: 30.7;
- Hausner ratio: 1.44.

Example 4 (comparative): preparation of monohydrate DF2755A by crystallization in presence of water (failed)

The preparation of Example 2A was repeated on a 100 g scale in the presence of water with the aim to directly obtain the monohydrate polymorph.

In particular, during the salification step, the solution was cooled at 20°C, and then toluene and 6% of water were added. After 1 hour, the product started to precipitate and the resulting solid was stirred for further 12 hours at 5°C.

However, the product obtained after filtration and drying under vacuum at 40°C for about 24 hours, was the anhydrous DF2755A. The structure was confirmed by $^1$H-NMR, HPLC, Karl-Fischer and XRPD analysis.

Example 5 (comparative): preparation of stable stoichiometric hydrates DF2755A by controlled hydration (failed)

Samples of anhydrous DF2755A, prepared according to Example 2A, were placed in a climatic chamber at the following conditions:

| | |
|---|---|
| Ex. 5A: | 24 hours, 25°C 50% R.H. |
| Ex. 5B: | 24 hours, 25°C 60% R.H. |
| Ex. 5C | 72 hours, 25°C 60% R.H. |

At the end of the hydration experiments, all the samples analyzed by $^1$H-NMR, HPLC, Karl-Fischer and XRPD resulted to be non-stoichiometric hydrates and not the monohydrate DF2755A.

## Claims

1. Crystalline monohydrate sodium (2S)-2-(4-{[4-(trifluoromethyl)-1,3-thiazol-2-yl] amino} phenyl) propanoate **charac-**

**terized by** an X-ray diffraction pattern (XRPD) with relevant peaks at 7.9, 18.3, 19.8, 23.8 and 25.5°2-theta ± 0.2 degrees 2-theta.

2. The crystalline monohydrate sodium (2S)-2-(4-{[4-(trifluoromethyl)-1,3-thiazol-2-yl] amino} phenyl) propanoate of claim 1 having a water content from 5.0% to 6.3% by weight, preferably from 5.0% to 5.5% by weight, more preferably from 5.0% to 5.3% by weight, measured by Karl Fischer method.

3. The crystalline monohydrate sodium (2S)-2-(4-{[4-(trifluoromethyl)-1,3-thiazol-2-yl] amino} phenyl) propanoate of claim 1 or 2 **characterized by** an X-ray diffraction pattern (XRPD) with further relevant peaks at 9.3, 15.5, 22.1, 22.3, 23.0 and 24.6° 2-theta ± 0.2 degrees 2-theta.

4. The crystalline monohydrate sodium (2S)-2-(4-{[4-(trifluoromethyl)-1,3-thiazol-2-yl] amino} phenyl) propanoate of any one of the previous claims **characterized by** one or more of the following powder properties:

   - particle size distribution of D(0.1) = 2 - 2.5 μm, D(0.5) = 6.5 - 8.5 μm, D(0.9) = 20 - 30 μm, measured by Malvern Mastersizer 3000 using Aero S accessory;
   - bulk density from 0.17 to 0.20 g/ml, measured according to Ph. Eur. 2.9.34;
   - tapped density from 0.19 g/ml to 0.28 g/ml, measured according to Ph. Eur. 2.9.34;
   - compressibility index from 23 to 33;
   - Hausner ratio lower than 1.50.

5. A composition comprising, consisting essentially of or consisting of the crystalline monohydrate according to any one of claims 1 to 4.

6. The composition of claim 5 with a content of (2S)-2-(4-{[4-hydroxy-4-(trifluoromethyl)-4,5-dihydro-1,3-thiazol-2-yl] amino}phenyl)propanoic acid lower than 0.5%, preferably lower than 0.2%, more preferably lower than 0.1% determined by HPLC method according to the description.

7. A process for the preparation of crystalline monohydrate sodium (2S)-2-(4-{[4-(trifluoromethyl)-1,3-thiazol-2-yl] amino} phenyl) propanoate comprising:

   i) providing anhydrous sodium (2S)-2-(4-{[4-(trifluoromethyl)-1,3-thiazol-2-yl] amino} phenyl) propanoate; and
   ii) exposing it to an atmosphere having a Relative Humidity (R.H.) higher than 60% and lower than 80% by weight, at a temperature from 25°C to 40 °C and at atmospheric pressure, preferably for a time from 12 to 72 hours, thus providing crystalline monohydrate sodium (2S)-2-(4-{[4-(trifluoromethyl)-1,3-thiazol-2-yl] amino} phenyl) propanoate.

8. The process of claim 7 wherein the atmosphere R.H. is from 65% to 75% by weight.

9. The process of claim 7 or 8 wherein the temperature is from 25°C to 30°C and, preferably, the time is from 24 to 72 hours.

10. The process of any one of claims 7 to 9 wherein the anhydrous sodium (2S)-2-(4-{[4-(trifluoromethyl)-1,3-thiazol-2-yl] amino} phenyl) propanoate of step i) is prepared according to the process of claim 11 or 12.

11. A process for the preparation of anhydrous sodium (2S)-2-(4-{[4-(trifluoromethyl)-1,3-thiazol-2-yl] amino} phenyl) propanoate comprising:

   - providing a solution of (2S)-2-(4-{[4-(trifluoromethyl)-1,3-thiazol-2-yl] amino} phenyl) propanoic acid in a solvent selected among ethyl acetate, isobutyl acetate, propyl acetate and admixtures thereof, in which the concentration of the acid in the solution is from 0.08 to 0.12 Kg/l,
   - adding to the solution a sodium base in a molar ratio compared to the acid from 0.9:1 to 0.95:1, thus providing an admixture,
   - adding to the admixture an anti-solvent selected among toluene, m-xylene, p-xylene and admixtures thereof, the anti-solvent being in a volumetric ratio compared to the solvent from 0.4:1 to 0.6:1, thus precipitating anhydrous sodium (2S)-2-(4-{[4-(trifluoromethyl)-1,3-thiazol-2-yl] amino} phenyl) propanoate.

12. The process of claim 11 wherein:

- the concentration of the (2S)-2-(4-{[4-(trifluoromethyl)-1,3-thiazol-2-yl] amino} phenyl) propanoic acid in the solution is from 0.09 to 0.11 Kg/l;
- the solvent is isobutyl acetate;
- the sodium base is sodium hydroxide, preferably aqueous sodium hydroxide;
- the anti-solvent is toluene;
- the volumetric ratio of the anti-solvent compared to the solvent is around 0.5:1; and
- the precipitation is carried out by cooling at temperatures lower than 20 °C, preferably lower than 10°C.

**13.** A pharmaceutical composition comprising a therapeutically effective amount of crystalline monohydrate sodium (2S)-2-(4-{[4-(trifluoromethyl)-1,3-thiazol-2-yl] amino} phenyl) propanoate of any one of claims 1 to 4 and at least a pharmaceutically acceptable excipient.

**14.** Crystalline monohydrate sodium (2S)-2-(4-{[4-(trifluoromethyl)-1,3-thiazol-2-yl] amino} phenyl) propanoate of any one of claims 1 to 4, for use as a medicament.

**15.** Crystalline monohydrate sodium (2S)-2-(4-{[4-(trifluoromethyl)-1,3-thiazol-2-yl] amino} phenyl) propanoate of any one of claims 1 to 4, for use in the prevention or treatment of an acute or chronic inflammatory-mediated disease, inflammatory and post-operative pain, interstitial cystitis (IC)/ bladder pain syndrome (BPS) and/or cancer.

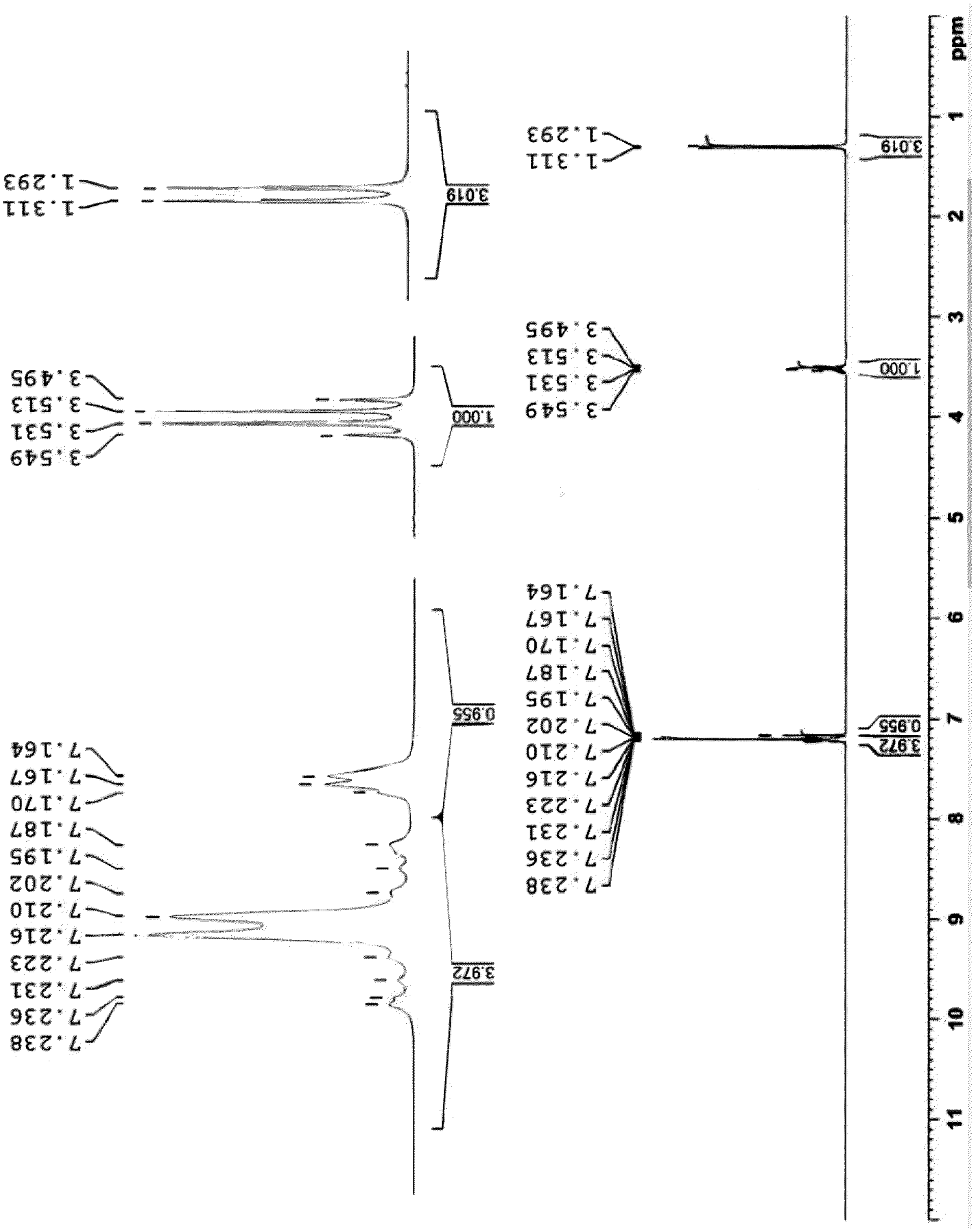
ppm
1.293
1.311
3.019
3.495
3.513
3.531
3.549
1.000
7.164
7.167
7.170
7.187
7.195
7.202
7.210
7.216
7.223
7.231
7.236
7.238
0.955
3.972

FIG.1

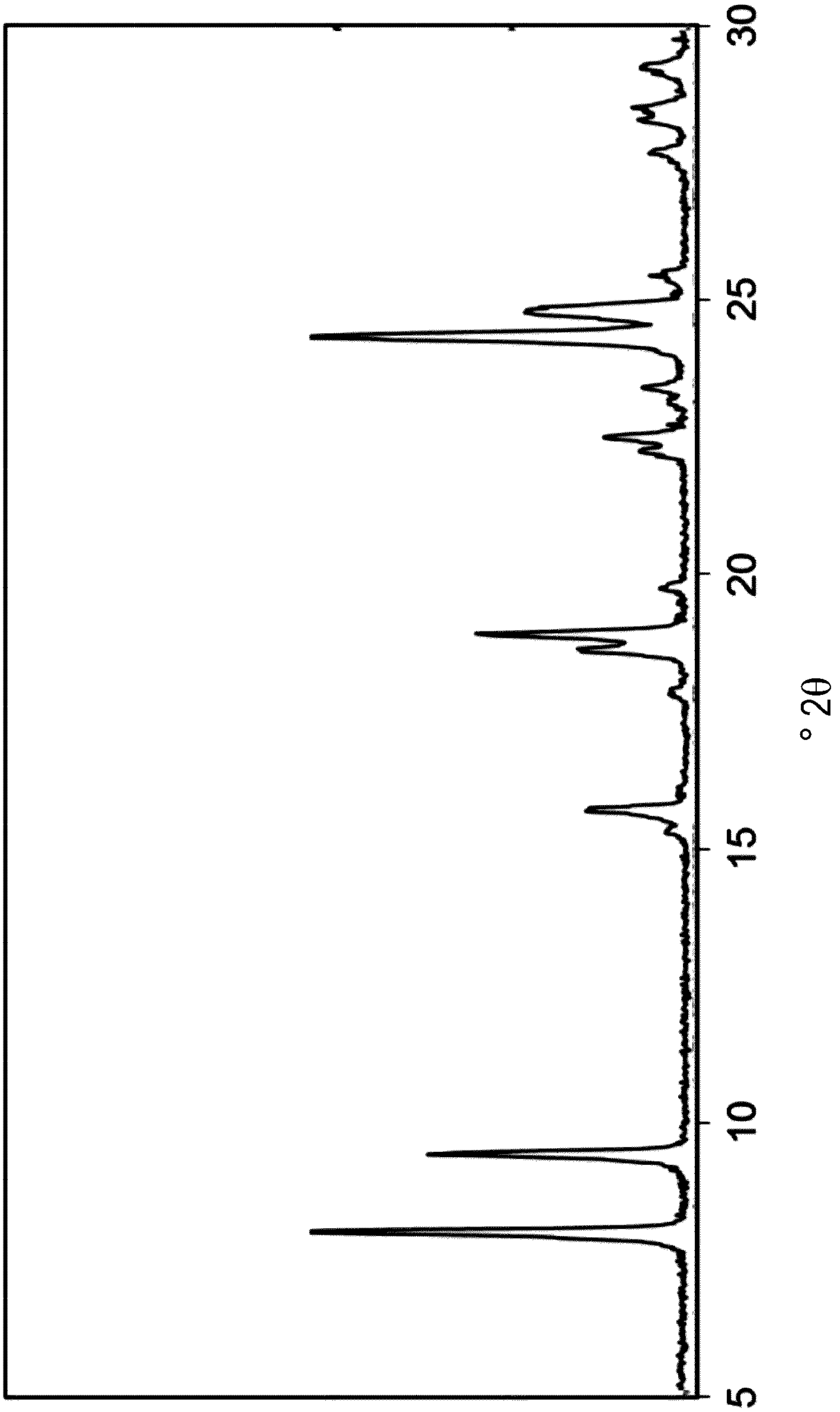


FIG.2

FIG.3

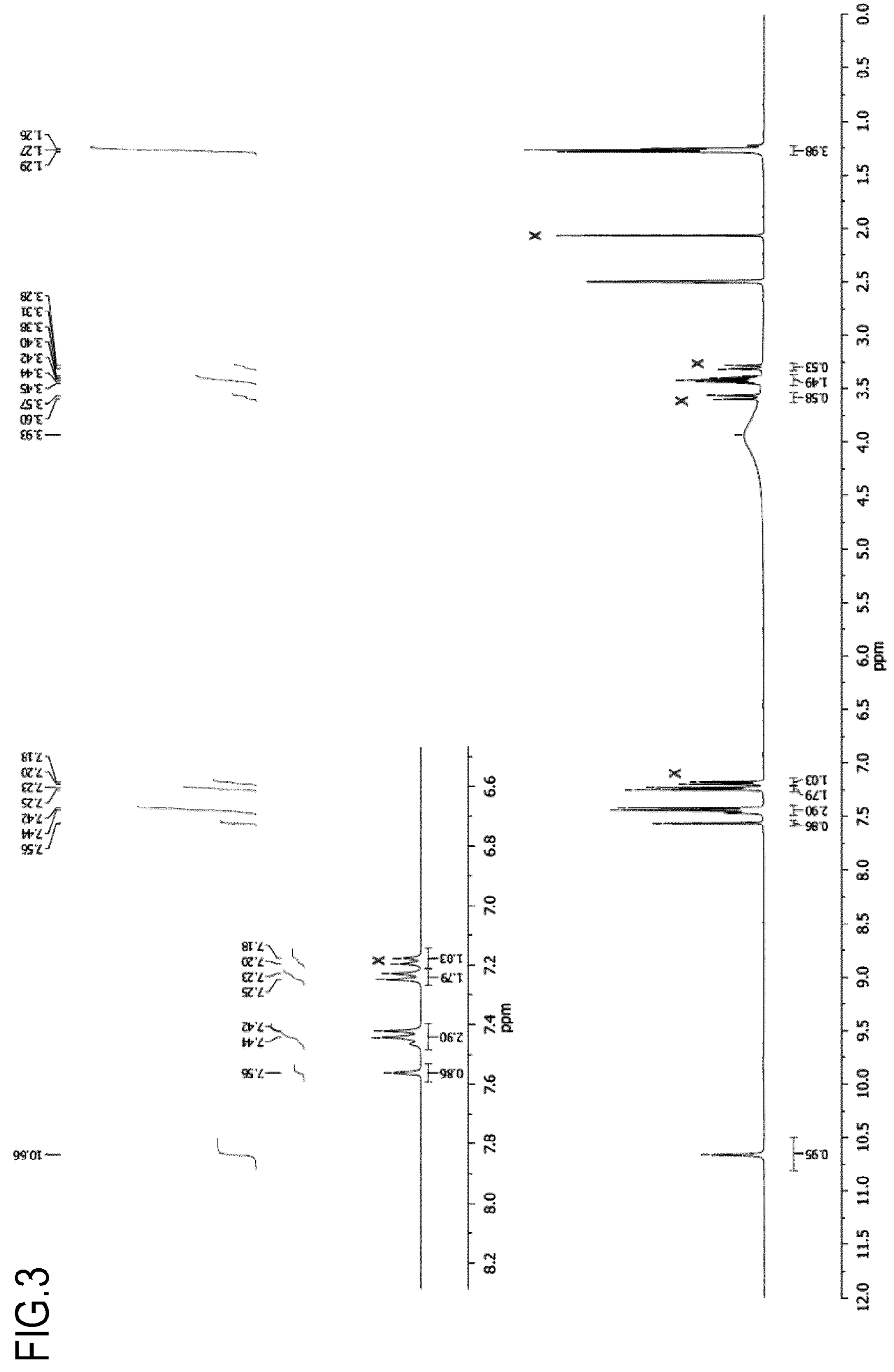
1.26
1.27
1.29
3.28
3.31
3.38
3.40
3.42
3.44
3.45
3.57
3.60
3.93
7.18
7.20
7.23
7.25
7.42
7.44
7.56
10.66
3.98
0.53
1.49
0.58
1.03
1.79
2.90
0.86
0.95
ppm

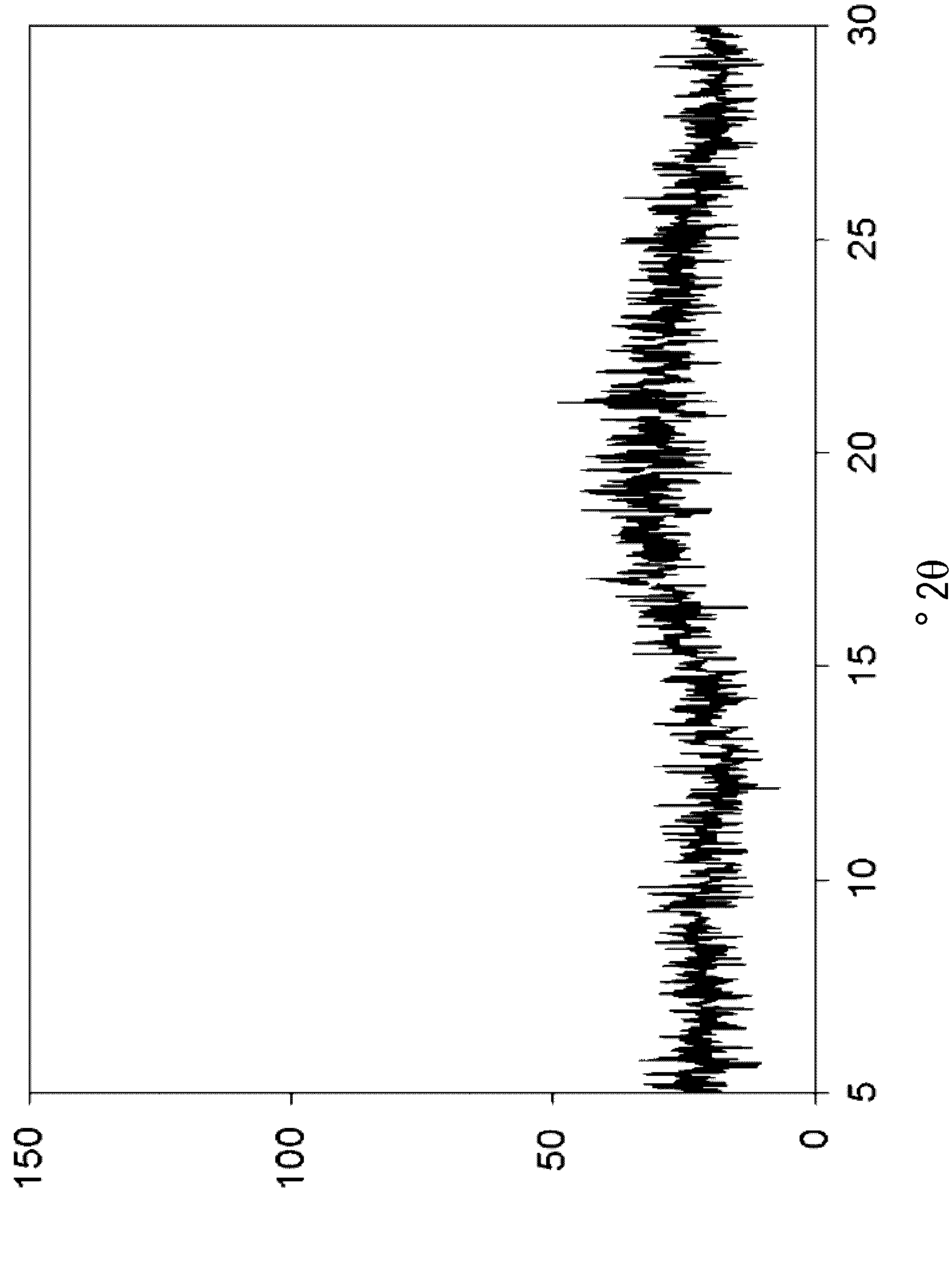


FIG.4

FIG.5

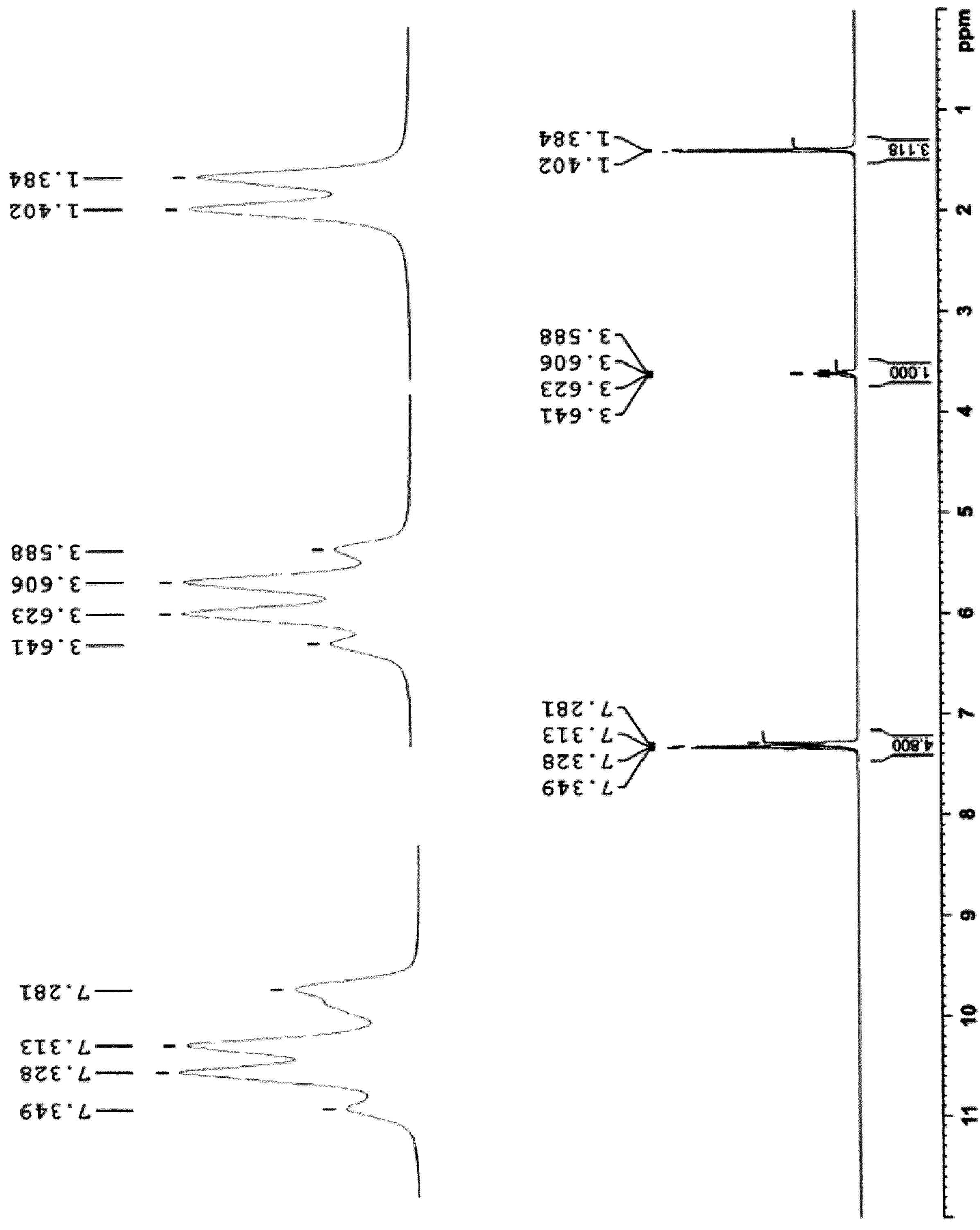
1.384
1.402
3.588
3.606
3.623
3.641
7.281
7.313
7.328
7.349
3.118
1.000
4.800
ppm

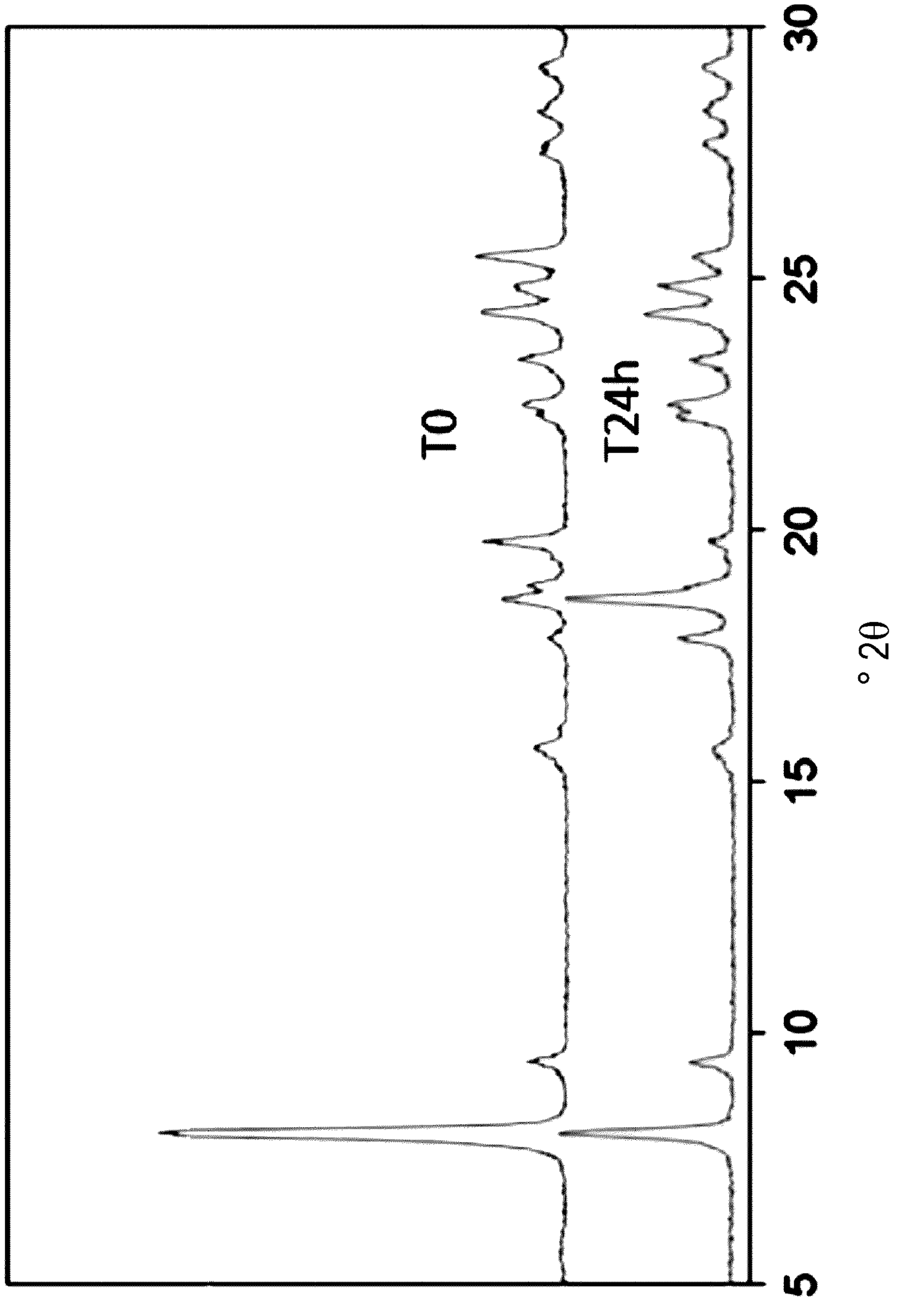


FIG.6

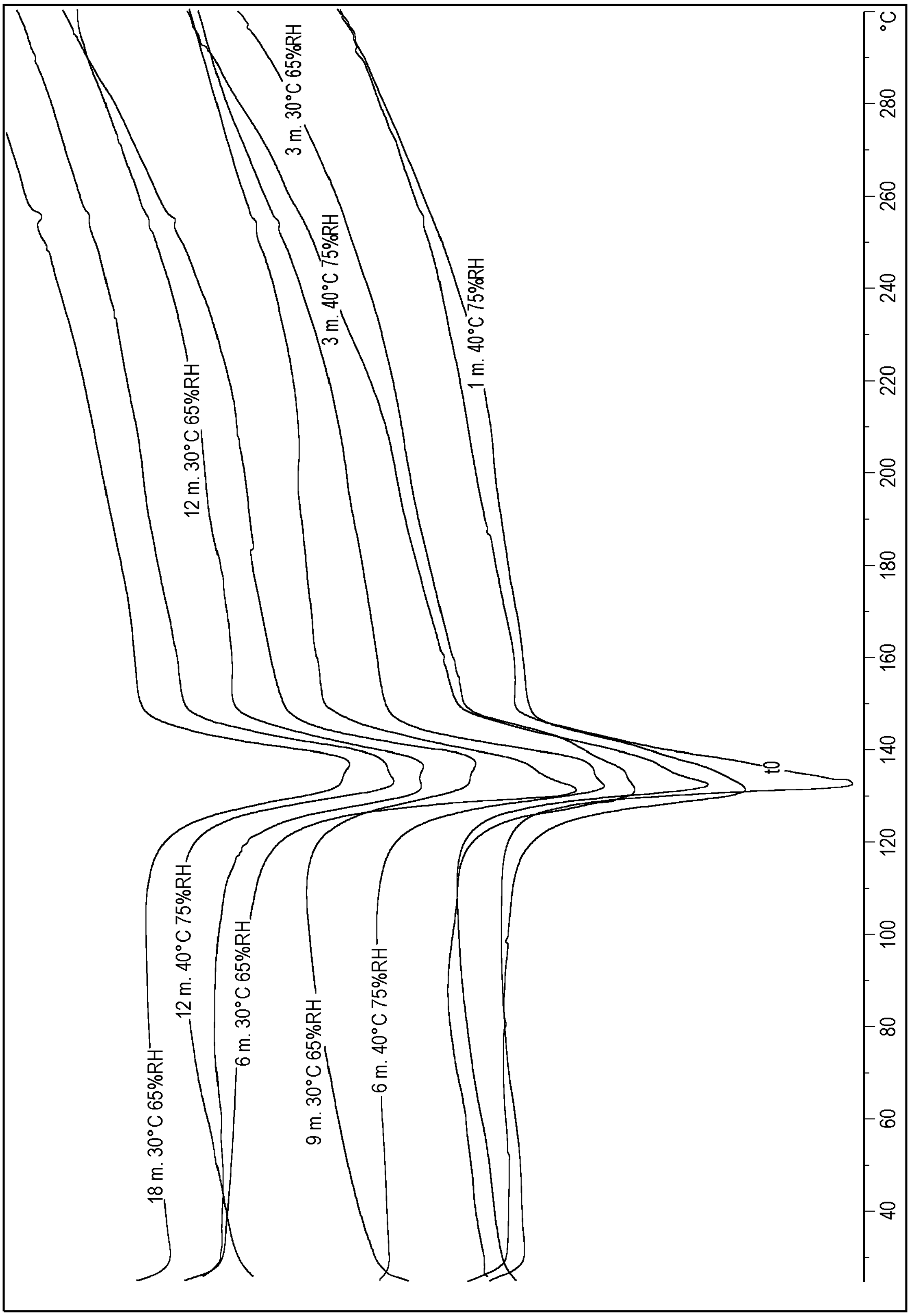
°C
40
60
80
100
120
140
160
180
200
220
240
260
280
18 m. 30°C 65%RH
12 m. 40°C 75%RH
6 m. 30°C 65%RH
9 m. 30°C 65%RH
6 m. 40°C 75%RH
12 m. 30°C 65%RH
3 m. 40°C 75%RH
3 m. 30°C 65%RH
1 m. 40°C 75%RH
t0

FIG.7

FIG.8

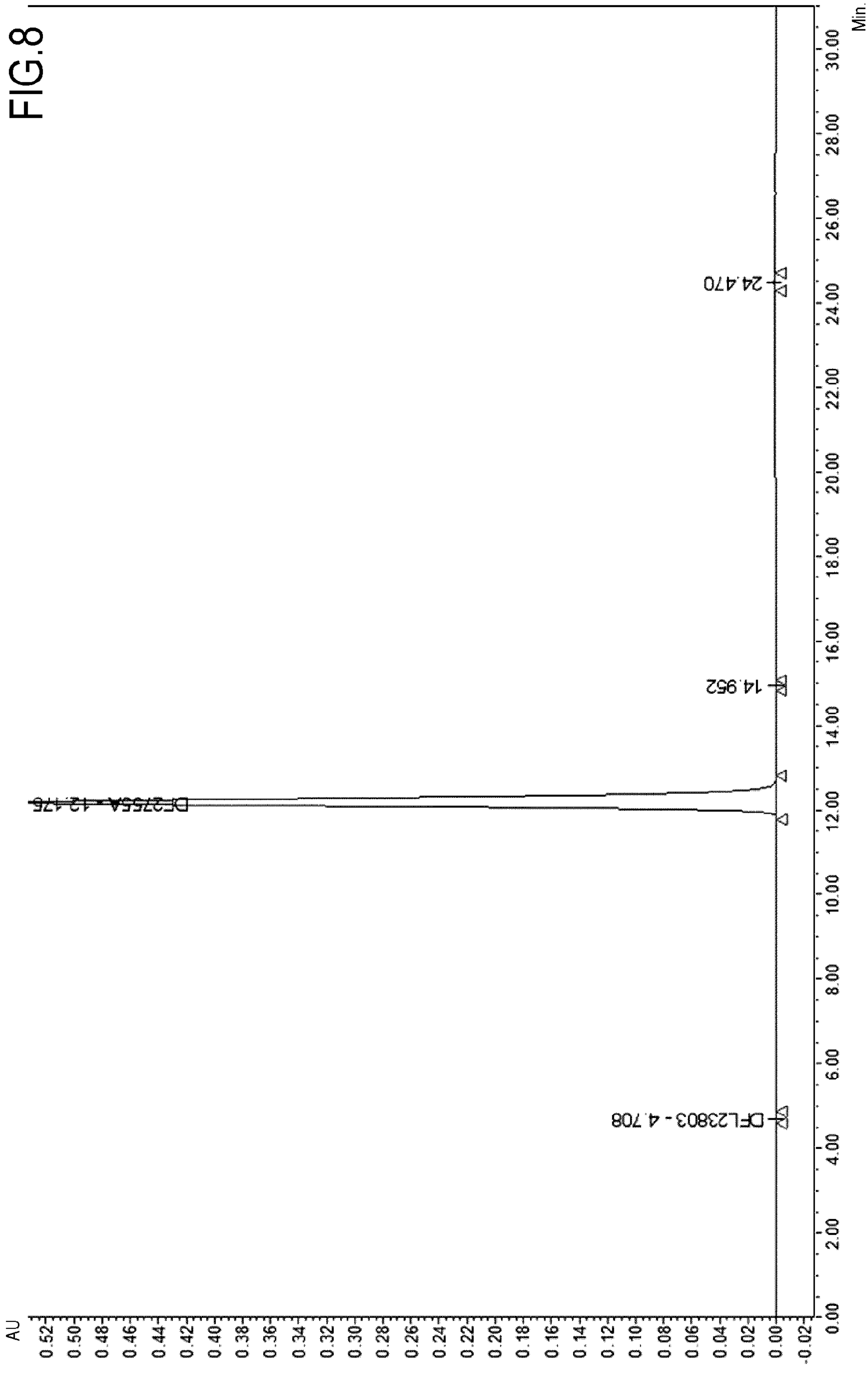

Europäisches Patentamt
European Patent Office
Office européen des brevets

# EUROPEAN SEARCH REPORT

Application Number
EP 23 15 3520

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 2 166 006 A1 (DOMPE SPA [IT]) 24 March 2010 (2010-03-24) * pages 9, 13; claims 1, 6-8, 10; compounds 3, 3a * ----- | 1-10, 13-15 | INV. C07D277/42 A61K31/426 A61P29/00 A61P35/00 |
| A | COLELLA MARCO ET AL: "1,3-Dibromo-1,1-difluoro-2-propanone as a Useful Synthon for a Chemoselective Preparation of 4-Bromodifluoromethyl Thiazoles", ACS OMEGA, [Online] vol. 3, no. 11, 30 November 2018 (2018-11-30), pages 14841-14848, XP093047276, US ISSN: 2470-1343, DOI: 10.1021/acsomega.8b02273 Retrieved from the Internet: URL:http://pubs.acs.org/doi/pdf/10.1021/acsomega.8b02273> [retrieved on 2023-05-16] * pages 14844 and 14847; compound DF2755Y * ----- | 1-10, 13-15 | TECHNICAL FIELDS SEARCHED (IPC) C07D A61P A61K |
| A | JURCZAK EWA ET AL: "Pharmaceutical Hydrates Analysis—Overview of Methods and Recent Advances", PHARMACEUTICS, [Online] vol. 12, no. 10, 11 October 2020 (2020-10-11), page 959, XP055802485, DOI: 10.3390/pharmaceutics12100959 Retrieved from the Internet: URL:https://www.ncbi.nlm.nih.gov/pmc/articles/PMC7601571/> [retrieved on 2023-05-16] * page 4 of 25, lines 2-3 * ----- | 1-10, 13-15 | |

~~The present search report has been drawn up for all claims~~

1

| Place of search | Date of completion of the search | Examiner |
|---|---|---|
| Munich | 16 May 2023 | Moriggi, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

Application Number
EP 23 15 3520

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-10, 13-15

☐ The present supplementary European search report has been drawn up for those parts of the European patent application which relate to the invention first mentioned in the claims (Rule 164 (1) EPC).

Europäisches Patentamt
European Patent Office
Office européen des brevets

# LACK OF UNITY OF INVENTION SHEET B

Application Number
EP 23 15 3520

The Search Division considers that the present European patent application does not comply with the requirements of unity of invention and relates to several inventions or groups of inventions, namely:

**1. claims: 1-10, 13-15**

**The crystalline monohydrate sodium salt of (2S)-2-(4-{[4-(trifluoromethyl)-1,3-thiazol-2-yl]amino} phenyl)propanoic acid, a composition containing it, a process for its preparation, a pharmaceutical composition containing it and its medical use**

---

**2. claims: 11, 12**

**A process for the preparation of an anhydrous sodium salt of (2S)-2-(4-{[4-(trifluoromethyl)-1,3-thiazol-2-yl]amino} phenyl)propanoic acid**

---

## ANNEX TO THE EUROPEAN SEARCH REPORT ON EUROPEAN PATENT APPLICATION NO. EP 23 15 3520

This annex lists the patent family members relating to the patent documents cited in the above-mentioned European search report. The members are as contained in the European Patent Office EDP file on
The European Patent Office is in no way liable for these particulars which are merely given for the purpose of information.

16-05-2023

| Patent document cited in search report | Publication date | Patent family member(s) | Publication date |
|---|---|---|---|
| EP 2166006 A1 | 24-03-2010 | AU 2009294558 A1 | 25-03-2010 |
| | | BR PI0918759 A2 | 29-12-2015 |
| | | CA 2737099 A1 | 25-03-2010 |
| | | CN 102159558 A | 17-08-2011 |
| | | CY 1116176 T1 | 08-02-2017 |
| | | DK 2346841 T3 | 20-04-2015 |
| | | EP 2166006 A1 | 24-03-2010 |
| | | EP 2346841 A2 | 27-07-2011 |
| | | ES 2534634 T3 | 27-04-2015 |
| | | HR P20150395 T1 | 19-06-2015 |
| | | IL 211683 A | 30-01-2014 |
| | | JP 5571669 B2 | 13-08-2014 |
| | | JP 2012502957 A | 02-02-2012 |
| | | PL 2346841 T3 | 31-07-2015 |
| | | PT 2346841 E | 07-05-2015 |
| | | RU 2011114992 A | 27-10-2012 |
| | | SI 2346841 T1 | 31-07-2015 |
| | | SM T201500098 B | 09-07-2015 |
| | | US 2011207785 A1 | 25-08-2011 |
| | | WO 2010031835 A2 | 25-03-2010 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2010031835 A2
- WO 2010031835 A

### Non-patent literature cited in the description


- *Theranostics,* 2017, vol. 7 (6), 1543-1588
- *Pharmacological Research,* 2016, vol. 103, 69-79
- **A.H. KIBBE.** Handbook of Pharmaceutical Excipients. American Pharmaceutical Association